(19) Europäisches Patentamt
European Patent Office
Office européen des brevets

(11) Veröffentlichungsnummer: **0 024 683**
**B1**

(12) # EUROPÄISCHE PATENTSCHRIFT

(45) Veröffentlichungstag der Patentschrift: **27.10.82**

(51) Int. Cl.³: **C 07 C 154/00**

(21) Anmeldenummer: **80104945.3**

(22) Anmeldetag: **20.08.80**

(54) **Verfahren zur Herstellung von Thiochlorformiaten.**

(30) Priorität: **28.08.79 DE 2934657**

(43) Veröffentlichungstag der Anmeldung: **11.03.81 Patentblatt 81/10**

(45) Bekanntmachung des Hinweises auf die Patenterteilung: **27.10.82 Patentblatt 82/43**

(84) Benannte Vertragsstaaten: **AT BE CH DE FR GB IT LI NL**

(56) Entgegenhaltungen:
**US-A-3 277 143**
**US-A-3 766 236**

(73) Patentinhaber: **HOECHST AKTIENGESELLSCHAFT, Postfach 80 03 20, D-6230 Frankfurt/Main 80 (DE)**

(72) Erfinder: **Semler, Günther, Dr., Johann-Strauss-Strasse 44, D-6233 Kelkheim (Taunus) (DE)**
Erfinder: **Schaeffer, Georg, Dr., Herderstrasse 58, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 024 683 B1

BUNDESDRUCKEREI BERLIN

# Verfahren zur Herstellung von Thiochlorformiaten

Thiochlorformiate sind wertvolle Zwischen- und Endprodukte auf zahlreichen Sachgebieten, insbesondere auf dem Gebiet der Pflanzenschutzmittel (Mittel gegen Fungi, Nematoden etc.).

Zur Herstellung von Thiochlorformiaten ist eine Reihe von Methoden bekannt. Eine gängige Methode besteht in der Umsetzung von Mercaptanen (worunter hier ganz allgemein organische HS-Verbindungen verstanden werden) mit Phosgen. Diese Methode verläuft aber nur dann in befriedigender Weise zu den gewünschten Thiochlorformiaten, wenn man dabei bestimmte spezifisch wirkende Katalysatoren verwendet.

Ein derartiger spezifisch wirkender Katalysator ist z. B. Aktivkohle (US-PS 3 039 537). Der Hauptnachteil dieses Katalysators besteht jedoch darin, daß er beträchtliche Mengen der Ausgangs- und Endprodukte adsorbiert. Das adsorbierte hochgiftige Phosgen führt dann bei der Abtrennung des Katalysators durch Filtration infolge teilweiser Desorption zu technischen Schwierigkeiten. Bei Verwendung von Festbettreaktoren kommt es durch das adsorbierte jeweilige Mercaptan und das entsprechende — ebenfalls adsorbierte — Thiochlorformiat zu erheblichen Problemen beim Produktwechsel — wenn also unter Verwendung desselben Katalysators ein anderes Thiochlorformiat hergestellt werden soll.

Die mit der Verwendung von Aktivkohle als Katalysator verbundenen Schwierigkeiten werden auch durch die spezielle zweistufige Verfahrensweise gemäß DE-OS 2 721 683 und DE-OS 2 809 776 nicht überwunden.

Eine Überwindung dieser in erster Linie durch Adsorption bedingten Schwierigkeiten ist dagegen möglich bei Verwendung anderer, nicht oder nur in geringem Maß adsorbierender Katalysatoren; als solche Katalysatoren sind insbesondere Carbonsäureamide (US-PS 3 277 143) und tertiäre Amine (US-PS 3 299 114) bekannt geworden.

Diese in einer Menge von 1 bis etwa 10 Mol-%, bezogen auf das Ausgangsmercaptan, eingesetzten Katalysatoren führen zwar — ebenso wie der Katalysator Aktivkohle — zu teilweise recht befriedigenden Thiochlorformiate-Ausbeuten (bis zu etwa 90%), doch ist eine Aufarbeitung der Reaktionsansätze durch Destillation ohne vorherige Entfernung des Katalysators nicht möglich (Zersetzungsreaktionen, Verfärbungen des Destillats, Sublimation von Feststoffen etc.). Die Katalysator-Entfernung geschieht durch Waschen mit Wasser oder wäßriger Salzsäure, wodurch in gewissem Ausmaß Hydrolyse der Thiochlorformiate eintritt, was sich auf Ausbeute und Qualität des gewünschten Endprodukts ungünstig auswirkt. Außerdem stellt insbesondere bei der Durchführung des Verfahrens in technischem Maßstab die Katalysator-Entfernung durch Auswaschen mit Wasser oder wäßriger Salzsäure einen das Verfahren verteuernden Kostenfaktor dar.

Wegen des ansonsten einfachen und günstigen Syntheseweges zu Thiochlorformiaten durch Umsetzung von Mercaptanen mit Phosgen war es daher wünschenswert und bestand die Aufgabe, die bekannten Verfahren weiter zu verbessern.

Diese Aufgabe konnte erfindungsgemäß in überraschender und einfacher Weise dadurch gelöst werden, daß man bei dem aus der US-PS 3 277 143 bekannten Verfahren geringere Katalysatormengen verwendet, wobei außer den dort als Katalysatoren genannten Carbonsäureamiden auch Derivate des Harnstoffs (= Amide der Kohlensäure) zum Einsatz kommen können.

Erfindungsgegenstand ist daher ein Verfahren zur Herstellung von Thiochlorformiaten durch Umsetzung von Mercaptanen mit Phosgen in Gegenwart mindestens eines Carbonsäureamids und/oder Harnstoffderivats als Katalysator, das dadurch gekennzeichnet ist, daß man den Katalysator in einer Menge von etwa 0,02 bis etwa 0,2 Mol-%, vorzugsweise von etwa 0,05 bis etwa 0,1 Mol-%, bezogen auf das Ausgangsmercaptan, verwendet.

Es ist außerordentlich überraschend, daß die Umsetzung mit diesen geringen Katalysatormengen genauso gut abläuft wie mit den aus der US-PS 3 277 143 bekannten höheren Mengen; denn nach dieser US-PS mußte man davon ausgehen, daß Katalysatormengen unter etwa 2 Mol-% (bezogen auf das Ausgangsmercaptan) nicht oder jedenfalls nicht in ausreichendem Maß wirksam sind, da normalerweise aus Wirtschaftlichkeitsgründen keine höheren Katalysator- und übrige Stoffmengen als nötig eingesetzt werden. Die erfindungsgemäße Verbesserung hat zur — in gleicher Weise überraschende — Konsequenz, daß sich die Reaktionsansätze ohne vorherige Entfernung des Katalysators durch Destillation aufarbeiten lassen, wobei qualitativ einwandfreie Produkte in hoher Ausbeute erhalten werden.

Als Ausgangsmercaptane können für das erfindungsgemäße Verfahren alle möglichen organischen Mercaptoverbindungen mit einer oder mit mehreren SH-Gruppen im Molekül verwendet werden. Repräsentativ hierfür sind die in den US-PSen 3 277 143 und 3 299 114 genannten Mercaptane. Insbesondere kommen Mercaptane der Formel

$$R-SH$$

infrage, worin

R = Alkyl, vorzugsweise mit 1–18, insbesondere mit 1–8 C-Atomen
Cycloalkyl, vorzugsweise mit 5–6, insbesondere mit 6 C-Atomen
Alkenyl, vorzugsweise mit 3–8, insbesondere mit 3–4 C-Atomen
Aryl, vorzugsweise Phenyl oder Naphthyl, insbesondere Phenyl
Aralkyl, vorzugsweise Benzyl
heterocyclische Reste, vorzugsweise Thienyl oder Furyl

} ggf. (1- oder mehrfach) substituiert durch inerte Substituenten oder durch die SH-Gruppe

Die inerten Substituenten dieser Reste R sind vor allem:

Halogen, vorzugsweise F, Cl, Br, insbesondere Cl
Alkoxy, vorzugsweise ($C_1-C_4$)-Alkoxy, insbesondere Methoxy
Aryloxy, vorzugsweise Phenoxy
Carboalkoxy, vorzugsweise Carbo-($C_1-C_4$)-alkoxy, insbesondere Carbomethoxy.

Außer für R = Alkyl kommen als inerte Substituenten auch noch Alkylreste, vorzugsweise mit 1 bis 4 C-Atomen, insbesondere $CH_3$, infrage.

Für den Fall, daß die Reste R auch durch eine oder mehrere SH-Gruppen substituiert sind, liegen entsprechende 2- und mehrwertige Mercaptane vor.

Folgende konkrete Mercaptane werden in beispielhafter Weise genannt:

Ethylmercaptan, n-Propylmercaptan, iso-Propylmercaptan, tert.Butylmercaptan, Amylmercaptan, n-Octylmercaptan, Cyclohexylmercaptan, Allylmercaptan, Benzylmercaptan, Thiophenol, o-, m- und p-Thiokresol, o-, m- und p-Chlorthiophenol, Dichlorthiophenole, Bromthiophenole, Fluorthiophenole, Methoxythiophenole, Ethoxythiophenole, Phenoxythiophenole, Carbomethoxythiophenole, α-Thionaphthol, β-Thionaphthol, Dithiohydrochinon.

Besonders bevorzugte Ausgangs-Mercaptane sind die ($C_1-C_8$)-Alkyl-Mercaptane sowie Phenylmercaptan und die Tolyl-mercaptane.

Katalysatoren für das erfindungsgemäße Verfahren sind Carbonsäureamide, die im wesentlichen auch in der US-PS 3 277 143 beschrieben sind und subst. Harnstoffe. Die Katalysatoren fallen insbesondere unter folgende allgemeine Formel:

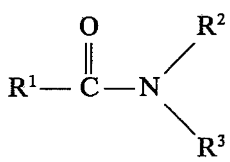

worin $R^1$ = H,
Alkyl, vorzugsweise mit 1–4 C-Atomen,
Phenyl, oder die Gruppe

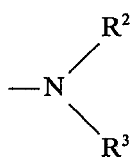

$R^2$ und $R^3$ = unabhängig voneinander H,
Alkyl, vorzugsweise mit 1–4 C-Atomen,
Phenyl,

wobei mindestens einer der Reste $R^1$, $R^2$ und $R^3$ ≠ H, wenn

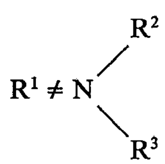

sowie mindestens einer der Reste $R^2$ und $R^3 \neq H$, wenn

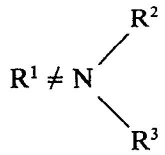

und 2 der Reste $R^1$, $R^2$, $R^3$ zusammen — also entweder $R^1+R^2$ oder $R^3$, oder $R^2+R^3$ — auch = Alkylen, vorzugsweise mit 3—6 C-Atomen, sein können.

Einige konkrete Katalysatoren sind in beispielhafter Weise:

Dimethylformamid
Dimethylacetamid
Diethylformamid
N,N-Diethylpropionsäureamid
Benzoyldimethylamid
Tetramethylharnstoff
Tetraethylharnstoff
Tetra-n-butylharnstoff
N-Methylpyrrolidon
N-Acetylpiperidin etc.

Besonders bevorzugte Katalysatoren sind Dimethylformamid, Tetramethylharnstoff und N-Methylpyrrolidon.

Die genannten Katalysatoren können sowohl einzeln als auch in praktisch beliebiger Mischung miteinander verwendet werden.

Es ist auch möglich die Katalysatoren in Form von deren (sich im Reaktionsansatz sowieso bildenden) Umsetzungsprodukten mit Phosgen oder dem jeweiligen Thiochlorformiat einzusetzen; die Umsetzungsprodukte sind — wenn man von Mercaptanen RSH, Phosgen $COCl_2$ und Katalysatoren

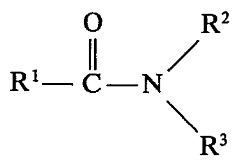

ausgeht — hauptsächlich Verbindungen der Formeln

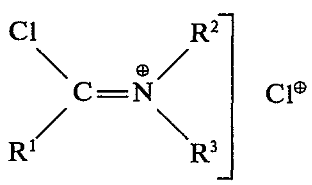

und wahrscheinlich

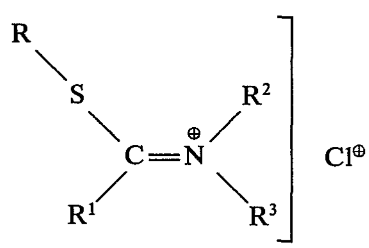

Die Verfahrensbedingungen für das erfindungsgemäße Verfahren im einzelnen entsprechen im wesentlichen den Bedingungen, welche in der US-PS 3 277 143 angegeben sind. Man arbeitet also hauptsächlich bei Raum- und nur möglichst wenig erhöhter Temperatur, da bei höheren Temperaturen Zersetzungen und Nebenreaktionen zunehmen. Als allgemeiner Temperaturbereich kann somit der Bereich zwischen etwa 20 und 80° C, insbesondere zwischen etwa 20 und 60° C, angewandt werden; bei beständigen Ausgangs- und Endprodukten kann im Einzelfall auch eine höhere Temperatur gewählt werden.

Die Reaktion wird im allgemeinen bei Normaldruck durchgeführt, wobei im besonderen Einzelfall u. U. auch unter Überdruck gearbeitet werden kann.

Das Verfahren wird zweckmäßig so ausgeführt, daß man Mercaptan und Katalysator vorlegt und laufend Phosgen durch das Reaktionsgemisch gast. In einer anderen Ausführungsform kann man auch ein Mercaptan-Katalysator-Gemisch und Phosgen gleichzeitig in das Reaktionsgemisch eindosieren. In dieser Form ist das Verfahren auch als kontinuierlicher Prozeß durchführbar.

Im Falle der Verwendung höher schmelzender Ausgangsmercaptane kann auch in den bei Phosgenierungsreaktionen üblichen Lösungsmitteln wie z. B. in Chlorbenzol etc. gearbeitet werden.

Nach Beendigung der Reaktion entfernt man das überschüssige gelöste Phosgen durch Ausblasen etwa mit Stickstoff und arbeitet den Ansatz im allgemeinen durch Destillation auf. Die Ausbeuten an Thiochlorformiaten liegen durchweg höher als die in der US-PS 3 277 143 angegebenen Ausbeuten.

Auch wegen der geringen Katalysatormengen und wegen des Wegfalls eines Arbeitsganges (Auswaschen) bei der Aufarbeitung des Reaktionsproduktes stellt das erfindungsgemäße Verfahren eine erhebliche Verbesserung der bekannten einschlägigen Verfahren und damit einen beträchtlichen Fortschritt dar.

Die Erfindung wird nun durch die folgenden Beispiele näher erläutert.

## Beispiel 1

### n-Octylthiochlorformiat

In einem Rührkolben mit Thermometer, Phosgeneinleitungsrohr und einem bei $-20°C$ betriebenen Rückflußkühler wird ein Gemisch aus 87,6 g n-Octylmercaptan und 0,09 g N-Methylpyrrolidon (=0,15 Mol-% bezogen auf n-Octylmercaptan) vorgelegt und bei 50°C so lange mit Phosgen behandelt, bis im Gaschromatogramm kein Ausgangsprodukt mehr nachweisbar ist und/oder ein verstärkter Phosgenrückfluß anzeigt, daß die Umsetzung beendet ist. Nach etwa einstündigem Nachrühren bei ebenfalls 50°C werden das überschüssige Phosgen und der restliche Chlorwasserstoff mittels Stickstoff ausgeblasen und die zurückbleibende hellgelbe Flüssigkeit im Vakuum destilliert (Kp.: 85°C/1,3 mbar). Man erhält 122,6 g (entspr. 98% d. Theorie) eines praktisch farblosen n-Octhylthiochlorformiates von 99%iger Reinheit (GC-Analyse).

## Vergleichsbeispiel (höhere Katalysatormenge)

Führt man die in Beispiel 1 beschriebene Reaktion unter Verwendung von 3 g N-Methylpyrrolidon (entspr. 5 Mol-%, bez. auf n-Octylmercaptan) durch, dann erhält man nur 94,0 g (entspr. 75,1% d. Theorie) eines stark gelb gefärbten n-Octylthiochlorformiates von 95%iger Reinheit (GC).

## Beispiele 2—7 mit Vergleichsbeispielen

Analog Beispiel 1 und dem zugehörigen Vergleichsbeispiel wurden weitere Beispiele (2—7) und Vergleichsbeispiele durchgeführt; Details s. die nachstehende Tabelle. In der Tabelle sind auch die entspr. Werte — soweit vorhanden — aus der Tabelle in Sp. 3/4 der US-PS 3 277 143 miteingetragen.

**Tabelle**

**$R\text{-}SH + COCl_2 \rightarrow R\text{-}S\text{-}COCl + HCl$**

| Bsp.: | R | Katalysator | Phosgenierungs-Temperatur (°C) | Ausb. an Thiochlorformiat (% d.Th.) | Siedepunkt °C/mbar | Reinheit % | Farbe |
|---|---|---|---|---|---|---|---|
| 2 | $n\text{-}C_8H_{17}-$ | 0,2 Mol-% DMF[1]) | 50 | 98,0 | 85/1,3 | 99,0 | fast farblos |
| Vgl. US-PS | $n\text{-}C_8H_{17}-$ | 3 Mol-% DMF[1]) | 50 | 77,8 | | 97,0 | gelb |
| | $n\text{-}C_8H_{17}-$ | 3 Mol-% DMF[1]) | | 86 | | – | – |
| 3 | $C_2H_5-$ | 0,1 Mol-% DMF[1]) | 35–50 | 88,2 | 82/230 | 98,7 | leicht gelb |
| Vgl. US-PS | $C_2H_5-$ | 3 Mol-% DMF[1]) | 35–50 | 75,3 | | 90,0 | tief gelb |
| | $C_2H_5-$ | 3 Mol-% DMF[1]) | | 79 | | – | – |
| 4 | $n\text{-}C_3H_7-$ | 0,07 Mol-% TMH[2]) | 50 | 93,2 | 67/40 | 98,7 | farblos |
| Vgl. US-PS | $n\text{-}C_3H_7-$ | 3 Mol-% TMH[2]) | 50 | 91,0 | | 98,0 | fast farblos |
| | $n\text{-}C_3H_7-$ | 3 Mol-% DECA[3]) | | 88 | | – | – |
| 5 | tert.-$C_4H_9-$ | 0,1 Mol-% DMF | 50 | 84,3 | 76/67 | | |
| Vgl. US-PS | tert.-$C_4H_9-$ | 7 Mol-% DMF | 50 | 51,4 | | | |
| | tert.-$C_4H_9-$ | 7 Mol-% DMF | | 66 | | – | – |
| 6 | $C_6H_5$ | 0,1 Mol-% DMF | 50 | 92,3 | 106/13 | 97,9 | gelb |
| Vgl. US-PS | $C_6H_5$ | 2,7 Mol-% DMF | 50 | 67,7 | | 94,2 | tief gelb, trüb |
| | $C_6H_5$ | 13,3 Mol-% DMF | | 68 | | – | – |
| 7 | $p\text{-}CH_3-C_6H_4-$ | 0,1 Mol-% DMF | 50 | 95,2 | 122/13 | 99,2 | gelb |
| Vgl. US-PS | $p\text{-}CH_3-C_6H_4-$ | 2,9 Mol-% DMF | 50 | 82,6 | | 99,0 | gelb, trüb |

[1]) = DMF = N,N-Dimethylformamid. [2]) = TMH = N,N,N',N'-Tetramethylharnstoff. [3]) = DECA = N,N-Diethylchloracetamid.

**Patentansprüche**

1. Verfahren zur Herstellung von Thiochlorformiaten durch Umsetzung von Mercaptanen mit Phosgen in Gegenwart mindestens eines Carbonsäureamids und/oder Harnstoffderivats als Katalysator, dadurch gekennzeichnet, daß man den Katalysator in einer Menge von 0,02 bis 0,2 Mol-%, vorzugsweise von 0,05 bis 0,1 Mol-%, bezogen auf das Ausgangsmercaptan, verwendet.

2. Verfahren nach Anspruch 1, dadurch gekennzeichnet, daß man als Mercaptane Verbindungen der Formel

RSH

verwendet, worin R folgende Bedeutung besitzt:

R = Alkyl, vorzugsweise mit 1–18, insbesondere mit 1–8 C-Atomen
Cycloalkyl, vorzugsweise mit 5–6, insbesondere mit 6 C-Atomen
Alkenyl, vorzugsweise mit 3–8, insbesondere mit 3–4 C-Atomen
Aryl, vorzugsweise Phenyl oder Naphthyl, insbesondere Phenyl
Aralkyl, vorzugsweise Benzyl
heterocyclische Reste, vorzugsweise Thienyl oder Furyl

} gegebenenfalls durch inerte Substituenten oder durch die SH-Gruppe substituiert

und als inerte Substituenten folgende Gruppen infrage kommen:

Halogen, vorzugsweise F, Cl, Br, insbes. Cl
Alkoxy, vorzugsweise ($C_1-C_4$)-Alkoxy, insbes. Methoxy
Aryloxy, vorzugsweise Phenoxy
Carboalkoxy, vorzugsweise Carbo-($C_1-C_4$)-alkoxy, insbes. Carbomethoxy,

sowie — außer im Falle R=Alkyl — auch Alkyl, vorzugsweise mit 1—4 C-Atomen, insbesondere Methyl.

3. Verfahren nach Ansprüchen 1—2, dadurch gekennzeichnet, daß man als Mercaptane ($C_1-C_8$)-Alkylmercaptane, Phenylmercaptan oder Tolylmercaptane verwendet.

4. Verfahren nach Ansprüchen 1—2, dadurch gekennzeichnet, daß man als Katalysatoren Verbindungen der Formel

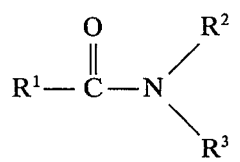

worin $R^1$ = H,
Alkyl, vorzugsweise mit 1–4 C-Atomen,
Phenyl, oder die Gruppe

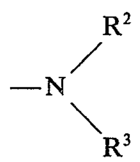

$R^2$ und $R^3$ = unabhängig voneinander H,
Alkyl, vorzugsweise mit 1–4 C-Atomen,
Phenyl,

wobei mindestens einer der Reste $R^1$, $R^2$ und $R^3$ ≠ H, wenn

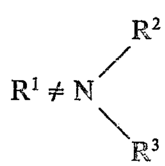

sowie mindestens einer der Reste $R^2$ und $R^3 \neq H$, wenn

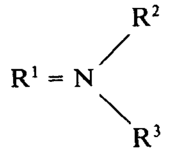

und 2 der Reste $R^1$, $R^2$, $R^3$ zusammen — also entweder $R^1+R^2$ oder $R^3$, oder $R^2+R^3$ — auch = Alkylen, vorzugsweise mit 3—6 C-Atomen; sein können.

5. Verfahren nach Ansprüchen 1—4, dadurch gekennzeichnet, daß man als Katalysatoren Dimethylformamid, Tetramethylharnstoff, und/oder N-Methylpyrrolidon verwendet.

**Claims**

1. A process for the preparation of thiochloroformates by reaction of mercaptans with phosgene in the presence of at least one carboxylic acid amide and/or urea derivative as catalyst, characterized in using the catalyst in an amount of from 0.02 to 0.2, preferably 0.05 to 0.1, mol-%, relative to the starting mercaptan.

2. The process as claimed in Claim 1, characterized in using as mercaptans compounds of the formula

$$R-SH,$$

in which

| | | |
|---|---|---|
| R | is alkyl, having preferably 1–18, especially 1–8 carbon atoms, cycloalkyl, having preferably 5–6, especially 6 carbon atoms, alkenyl, having preferably 3–8, especially 3–4 carbon atoms, aryl, preferably phenyl or naphthyl, especially phenyl, aralkyl preferably benzyl, a heterocyclic radical, preferably thienyl or furyl | optionally substituted by inert substitutents or the SH group |

whereby as inert substituents the following groups may be used:

halogen, preferably F, Cl, Br, especially Cl,
alkoxy, preferably $(C_1-C_4)$-alkoxy, especially methoxy,
aryloxy, preferably phenoxy,
carboalkoxy, preferably carbo-$(C_1-C_4)$-alkoxy, especially carbomethoxy,

and — except in the case that R = alkyl — also alkyl, preferably having 1—4 C-atoms, especially methyl.

3. The process as claimed in Claims 1 and 2, characterized in using as mercaptans $(C_1-C_8)$-alkylmercaptans, phenylmercaptan or tolylmercaptans.

4. The process as claimed in Claims 1 and 2, characterized in using as catalysts compounds of the formula

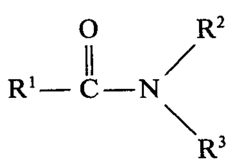

in which $R^1$ is H, alkyl having preferably from 1 to 4 carbon atoms, phenyl or the group

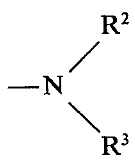

$R^2$ and $R^3$ independently from each other, are H, alkyl preferably having from 1 to 4 carbon atoms, phenyl, with the proviso that

at least one of the radicals $R^1$, $R^2$ and $R^3 \neq H$ when

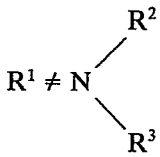

and at least one of the radicals $R^2$ and $R^3 = H$ when

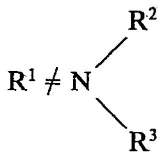

and two of the radicals $R^1$, $R^2$, $R^3$ together, that is either $R^1 + R^2$ or $R^3$, or $R^2 + R^3$, may furthermore be alkylene, preferably having from 3 to 6 carbon atoms.

5. The process as claimed in claims 1 to 4, characterized in using as catalysts dimethyl formamide, tetramethyl urea and/or N-methylpyrrolidone.

**Revendications**

1. Procédé de préparation de chlorothioformiates par réaction de mercaptans avec le phosgène en présence, comme catalyseur, d'au moins un carboxamide et/ou d'un dérivé de l'urée, procédé caractérisé en ce qu'on utilise le catalyseur en une quantité de 0,02 à 0,2% en moles, de préférence de 0,05 à 0,1% en moles par rapport au mercaptan de départ.

2. Procédé selon la revendication 1 caractérisé en ce qu'on utilise, comme mercaptans, des composés répondant à la formule:

$R-SH$

dans laquelle R représente un alkyle, de préférence en $C_1-C_{18}$, plus spécialement en $C_1-C_8$, un cycloalkyle, de préférence en $C_5$ ou $C_6$, plus spécialement en $C_6$, un alcényle, de préférence en $C_3-C_8$, plus spécialement en $C_3$ ou $C_4$, un aryle, de préférence un phényle ou un naphthyle, plus spécialement un phényle, un aralkyle, de préférence un benzyle, ou un radical hétérocylique, de préférence un thiényle ou un furyle, chacun de ces radicaux pouvant porter un groupe $-SH$ ou des substituants inertes, tels qu'un halogène, de préférence F, Cl ou Br, plus spécialement Cl, un alcoxy, de préférence un alcoxy en $C_1-C_4$, plus spécialement méthoxy, un aryloxy, de préférence un phénoxy, un alcoxycarbonyle, de préférence un alcoxycarbonyle dont la partie alcoxy contient de 1 à 4 atomes de carbone, plus spécialement un méthoxycarbonyle, ou (sauf dans le cas où R est lui-même un alkyle) un alkyle, de préférence en $C_1-C_4$-, plus particulièrement un méthyle.

3. Procédé selon l'une des revendications 1 et 2, caractérisé en ce qu'on utilise, comme mercaptans, des alcane-thiols en $C_1-C_8$, le phénylmercaptan ou les tolylmercaptans.

4. Procédé selon l'une des revendications 1 et 2, caractérisé en ce qu'on utilise, comme catalyseurs, des composés répondant à la formule:

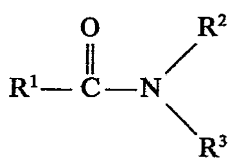

dans laquelle

$R^1$ représente l'hydrogène, un alkyle, de préférence un alkyle en $C_1$–$C_4$, un phényle ou un radical

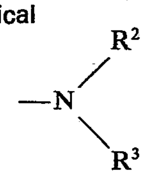

et

$R^2$ et $R^3$ représente chacun, indépendamment l'un de l'autre, H, un alkyle, de préférence un alkyle en $C_1$–$C_4$, ou un phényle,

au moins l'un des symboles $R^1$, $R^2$ et $R^3$ n'étant pas l'hydrogène dans le cas où $R^1$ n'est pas un radical

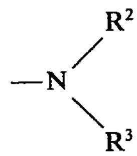

au moins l'un des symboles $R^2$ et $R^3$ n'étant pas l'hydrogène dans le cas où $R^1$ est un radical

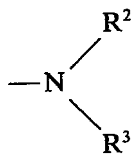

et deux des symboles $R^1$, $R^2$ et $R^3$ pris ensemble, donc soit $R^1+R^2$, soit $R^1+R^3$, soit $R^2+R^3$, pouvant également former un alkylène, de préférence un alkylène en $C_3-C_6$.

5. Procédé selon l'une quelconque des revendications 1 à 4, caractérisé en cen qu'on utilise, comme catalyseurs, le diméthylformamide, la tétraméthylurée et/ou la N-méthylpyrrolidone.